# EUROPEAN PATENT APPLICATION

(11) **EP 1 865 458 A1**
(43) Date of publication of application: **12.12.2007**
(21) Application number: 07011139.8
(22) Date of filing: 06.06.2007
(51) Int. Cl.: G06Q 50/00, G06F 19/00

(54) **Medical image management method for managing medical images, and medical image management apparatus and report preparation method using the same**

(30) Priority: 07.06.2006 JP 2006159121
(71) Applicant: OLYMPUS MEDICAL SYSTEMS CORP., Shibuya-ku Tokyo 151-0072 (JP)
(72) Inventor: Sugiyama, Astushi, Tokyo 186-0011 (JP); Sato, Musashi, Tokyo 196-0022 (JP)
(74) Representative: von Hellfeld, Axel

(57) **Abstract**

A first storage (66) stores a first medical image, positional information in the first medical image and regional information corresponding to the positional information, respectively. A second storage (68) stores second medical images. A display unit (50) displays the first medical image and the second medical images stored in the second storage (68). A selector (54) selects a second medical image to be associated with the first image. A specifying unit (56) which specifies a position to be associated with the selected second medical image and a position on the displayed first medical image. An extraction unit (60) extracts regional information associated with the specified position, based on the specified position and the positional information. A third storage (70) stores association between the extracted positional information and the selected second medical image.

## Description

The present invention relates to a medical image management method and, more particularly, to a medical image management method for managing acquired medical images, and a medical image management apparatus and a report preparation method using the same.

There have been systems that enable the inputting and referencing of information along the work flow of endoscopic examination. Such systems allow the input of information on examinations performed and the use of the inputted performance information in accounting procedures. Such conventional systems also make it possible to input a report in such a manner that a typical image out of the recorded images can be attached to the report information as an image representative of the results of the examination. Typically, a report input is made in three columns, namely, the findings column, the diagnosis column, and the treatment column, and a physician or the like in charge makes necessary entries in each of the columns. Consequently, even when there are findings, diagnoses, and treatments for a plurality of organs (regions), it is necessary to follow a procedure of first inputting the findings on all the organs, then recording the diagnoses of all the organs, and finally recording the treatments performed. In other words, the physician in charge specifies the regions concerned and enters the findings, diagnoses and treatments therefor. Also, such systems make it possible to attach medical images obtained in the examinations to the report.

### Related Art List

(1) Japanese Patent Application Laid-Open No. 2006-119855.

For example, when a report on an endoscopic examination of bronchial tubes is to be produced, there are normally not a few medical images prepared to be attached thereto in correspondence to a number of regions of the bronchial tubes. Also, since some medical images may look exactly like the others, it is importance to clearly define the correspondence between medical images and specific regions of the whole structure of the bronchial tube. It is desirable that this correspondence processing be easy when associating such medical images to be attached with a plurality of regions concerned.

The present invention has been made under the foregoing circumstances, and a general purpose thereof is to provide a technology for easily associating medical images to be attached with corresponding regions.

In order to resolve the above problems, a medical image management apparatus according to one embodiment of the present invention comprises: a first storage which stores a first medical image, positional information in the first medical image and regional information corresponding to the positional information, respectively; a second storage which stores a second medical image different from the first medical image stored in the first storage; a display unit which displays the first medical image stored in the first storage and the second medical image stored in the second storage in separate areas, respectively; a selector which selects a second medical image to be associated with the first image from among second images displayed on the display unit; a specifying unit which specifies a position to be associated with the second medical image selected by the selector wherein the position is a position on the first medical image displayed on the display unit; an extraction unit which extracts regional information associated with the specified position, based on the position specified by the specifying unit and the positional information stored in the first storage; and a third storage which stores association between the positional information extracted by the extraction unit and the second medical image selected by the selector.

The "first medical image" and the "second medical image" (or a plurality of "second medical images") may represent images displayed on the display unit or may represent image data. According to this embodiment, the positional information and the regional information are stored in such a manner that the positional information and the regional information are associated with each other. Thus, the regional information and the second medical image or second medical images can be easily associated with each other by merely specifying a position on the displayed first medical image as a position in which the second medical image is to be associated.

The third storage may further store association among the position specified by the specifying unit, the extracted positional information and the selected second medical image; and based on the positional association stored in the third storage the display unit may display additional information, indicating that the second medical image is associated therewith, on the first medical image. In such a case, the additional information indicating that the second medical image is associated therewith is displayed on the first medical image, thus enhancing the visibility.

The apparatus may further comprise a change/deletion unit which changes or deletes the association stored in the third storage by specifying an operation of changing or deleting the additional information displayed on the display unit. In such a case, the stored association is changed or deleted by changing or deleting the additional information, so that the change or deletion processing can be carried out with ease.

When the extraction fails to extract region information, the display unit displays a message indicating that extraction has failed. In this case, the message indicating the fact that the extraction has failed, the accurate and proper processing can be carried out.

The third storage may further store information on a medical treatment associated with the regional information; and the display unit may display information on a medical treatment stored in the third storage in an area different from the area in which the first medical image and the second medical image are displayed. In this case, the second medical image and the information on a medical treatment can be associated with one unit of regional information.

Another embodiment of the present invention relates to a method for managing medical images. This method comprises: displaying a first medical image and a second medical different from the second image on separate areas, respectively; selecting a second medical image to be associated with the first image from among second images displayed; specifying a position to be associated with the selected second medical image wherein the position is a position on the first medical image displayed; extracting regional information associated with the specified position, based on the positional information stored in the first storage, by referring to the positional information stored beforehand and regional information corresponding to the positional information; and associating the extracted regional information with the selected second medical image.

Still another embodiment of the present invention relates also to a method for managing medical images. This method is characterized by a feature that regional information associated beforehand with positional information specified on a displayed schematic image and an examination image to be compared with the regional information are stored in a recording medium in a manner such that the regional information and the examination image are brought into correspondence with each other. Hereinafter, a "schematic image" will also be referred to as a "schema image" and vice versa.

Still another embodiment of the present invention relates to a method for preparing a report. This method comprises: displaying a schematic image on a report entry screen, based on a specification from a person who prepares the report; specifying a region, where information on a medical treatment is to be inputted, on the displayed schematic image; displaying information on the specified region, as information to be inputted; prompting an input of the information on a medical treatment to the displayed information to be inputted; and preparing the report based on the inputted information on a medical treatment.

According to this embodiment, the information on medical treatments and the schematic images are easily brought into correspondence with each other, so that the report can be prepared with ease.

It is to be noted that any arbitrary combination of the above-described structural components or rearrangement in the form among a method, an apparatus, a system, a recording medium, a computer program and so forth are all effective as and encompassed by the present embodiments.

Embodiments will now be described by way of examples only, with reference to the accompanying drawings which are meant to be exemplary, not limiting.
FIG. 1 illustrates a hardware configuration of an endoscopic examination management system according to an embodiment of the present invention;
FIG. 2 illustrates a software configuration of an endoscopic examination management system shown in FIG. 1;
FIG. 3 illustrates a structure of a medical image management apparatus which is included in an endoscopic examination management system shown in FIG. 1;
FIG. 4 is a flowchart showing a procedure for managing medical images by a medical image management apparatus shown in FIG. 3;
FIG. 5 illustrates a report selection screen displayed on a monitor, shown in FIG. 3, for selecting a type of report to be inputted;
FIG. 6 shows a data structure of positional information and regional information stored in a first storage shown in FIG. 3;
FIG. 7 illustrates a report entry screen displayed on a monitor of FIG. 3;
FIG. 8 illustrates an observation range setting screen displayed on a monitor of FIG. 3;
FIG. 9 illustrates a detailed region setting screen displayed on a monitor of FIG. 3;
FIG. 10 illustrates a verification dialog for a detailed region displayed on a monitor of FIG. 3;
FIG. 11 illustrates a report entry screen displayed on a monitor of FIG. 3;
FIG. 12 illustrates another report entry screen displayed on a monitor of FIG. 3;
FIG. 13 shows a data structure for association stored in a third storage of FIG. 3;
FIG. 14 illustrates a completed screen after data entry displayed on a monitor of FIG. 3;
FIG. 15 illustrates another completed screen after data entry displayed on a monitor of FIG. 3;
FIG. 16 illustrates still another completed screen after data entry displayed on a monitor of FIG. 3; and
FIG. 17 illustrates a report prepared by a medical image management apparatus of FIG. 3.

The invention will now be described by reference to the preferred embodiments. This does not intend to limit the scope of the present invention, but to exemplify the invention.

An outline of the present invention will be given before a specific description thereof. Preferred embodiments of the present invention relate to a medical image management apparatus for managing medical images taken in an endoscopic examination (hereinafter referred to as examination images). For instance, when the object of an endoscopic examination is bronchial tubes, one time of endoscopic examination produces a plurality of examination images. The plurality of examination images represent various regions of the bronchial tubes. Therefore it' is desirable that the examination images can be easily associated with the regions whose images have been picked up. A medical image management apparatus according to the present embodiment, which stores a schema diagram (hereinafter also referred to as "schematic diagram") depicting the whole structure of the bronchial tubes, can display it and a plurality of examination images on the monitor at the same time. Note that coordinates are predefined on the schematic diagram and the correspondence of the regions of the bronchial tubes to the coordinates is also predefined. Hereinafter, the coordinates defined on the schematic diagram are referred to as "positional information", and the information on regions associated with the coordinate information "regional information". As a physician in charge selects and drag-and-drops an examination image on a point of the schematic diagram, the medical image management apparatus identifies the corresponding regional information from the drag-and-dropped positional information and establishes an association between the identified regional information and the examination image.

Also, in preparing a report, it is necessary to select a region or organ of interest in the process of entering the descriptions of findings, diagnosis, and treatment. Also, separate from the preparation of a report, it is necessary to select a region or organ of interest in the process of setting examination images in the report. Thus, the work of selecting the region or organ of interest must be repeated, which results in lowered working efficiency. It is desirable therefore that the region or organ selecting work be performed more efficiently. A medical image management apparatus according to the preferred embodiments of the present invention instructs the user to enter findings or the like concerning the regional information once the association between the regional information and the examination image is established through the aforementioned procedure. And as the physician in charge enters his/her findings or the like in response to the instruction, the findings or the like are also associated with the regional information and examination image.

It is to be noted that problems addressed by the present embodiment and an objective thereof may also be described as follows. The diagnostic information managed by an endoscopic electronic chart includes a region about which findings, diagnosis, and treatment entries are made. If this region is registered, it is possible to identify which of the regions the findings, diagnosis, and treatment are about. Conventional systems allow a region to be registered by selecting the region from a hierarchical glossary. A problem with such a system is that the hierarchy must be expanded to register the region. There is also a problem that another step must be taken to register which part of a schematic diagram (schematic representation of an organ) the registered region belongs to. Therefore, an object of a medical image management apparatus according to the present embodiment is to simplify the registration of a region by carrying out the registration of the region and the registration of which part of a schematic diagram the registered region belongs to in a single step.

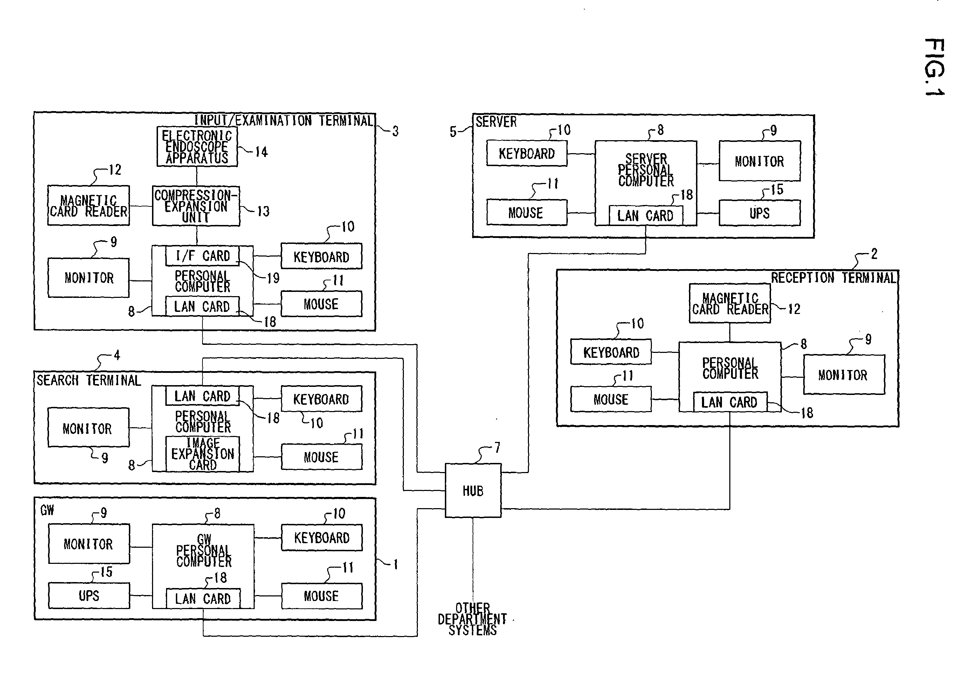

FIG. 1 illustrates a hardware configuration of an endoscopic examination management system according to an embodiment of the present invention. The endoscopic examination management system is comprised mainly of a GW (gateway) 1 for performing data exchange with other departments in a hospital, a reception terminal 2 for receiving applications for an examination, an input/examination terminal 3 to be used in performing an examination, a search terminal 4 for displaying or inputting images and various other information, and a server 5 for recording data. All the constituent terminals are connected with one another by a network (LAN) which is spread out from a HUB 7.

Each terminal, which is comprised basically of a personal computer 8, a monitor 9, a keyboard 10 and a mouse 11, is connected to the network via a LAN card 18 installed on the personal computer 8. The GW 1 includes a UPS (uninterruptible power supply) 15 in addition to the above-mentioned basic terminal composition and is therefore capable of coping with accidental power interruptions. The reception terminal 2 includes a magnetic card reader 12 in addition to the above-mentioned basic terminal composition. The reception terminal 2, which is used to receive applications for examination, is provided at a reception desk or the like in each department.

The input/examination terminal 3 includes a magnetic card reader 12, a compression-expansion unit 13, and an electronic endoscope apparatus 14 in addition to the above-mentioned basic terminal composition. The compression-expansion unit 13 is connected to the electronic endoscope apparatus 14 via an image cable and a communication cable. The compression-expansion unit 13 is connected to the personal computer 8 via an I/F card 19 installed in the personal computer 8. This input/examination terminal 3, which is used at the time of examination, is installed in the examination room.

The search terminal 4 has the above-mentioned basic terminal composition only. Since it is often used in conference, the search terminal 4 is placed in a conference room or the like. The server 5, which is placed in a server room, includes a UPS 15, in preparation for accidental power interruptions, in addition to the above-mentioned basic terminal composition. Note that a plurality of network HDDs to be connected to the server 5 via the HUB 7 may be provided for data recording. In such an arrangement, unique medium numbers are assigned to the plurality of network HDDs, respectively, and the server 5 manages the individual network HDDs.

Although not shown in detail, the electronic endoscope apparatus 14, which is similar to generally-known electronic endoscope apparatuses, is comprised of an electronic endoscope and a processor that is used to process and output image signals. The electronic endoscope is provided with a long and thin insertion unit which is to be inserted into the body and an operation unit attached to the rear end of the insertion unit. The operation unit is provided with a release switch for giving release instructions, and it is possible to record images by pressing this release switch.

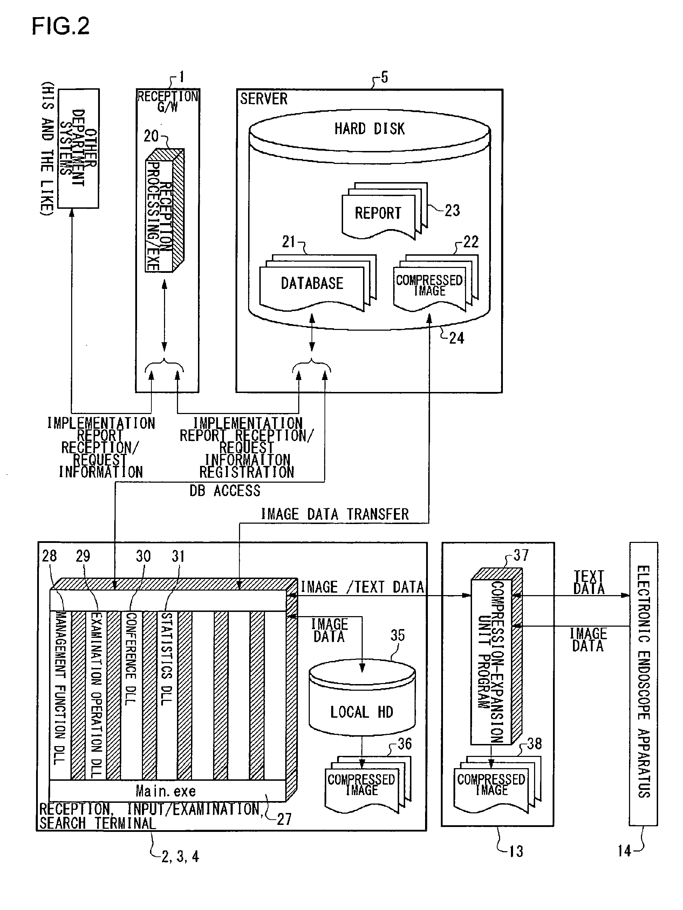

FIG. 2 illustrates a software configuration of an endoscopic examination management system. Various application software, database, etc., are installed to operate in the hard disk of each terminal. A GW application software 20 runs on the GW1.

In a hard disk 24 on the server 5, a database 21 operates and compressed images 22 picked up by the input/examination terminal 3 and report information 23 are recorded and stored. The server 5 has a function of sending a message of server fault to the other terminals when it has developed some trouble. Such troubles may include a full use of disk capacity, for instance. Since no operator is, as a rule, stationed in the server room, it is necessary that the server 5 detects a server fault by itself and communicates it to the other terminals.

At this time, it is preferable that if the network between the server 5 and the other terminals is offline, the server 5 stores the message until the network is restored and sends the message when it comes online. In this manner, any trouble with the server can be communicated to the operators of the terminals, and they can take prompt action in response to it.

Note that where there are a plurality of network HDDs for data accumulation, the server 5 may store the various data it handles in the network HDDs. In such an arrangement, the server 5 monitors the fault information, remaining capacity information, and the like of the network HDDs periodically using their assigned medium numbers. When some trouble arises with any network HDD, the server 5 sends a message of HDD fault to the other terminals. And if the connection to the other terminals is offline at this time, it is preferable that the server 5 sends the message of HDD fault to the other terminals only when an online status is recovered.

The reception terminal 2, the input/examination terminal 3, and the search terminal 4 share the same software composition, which is to be used by physicians and nurses in such a manner as to attain their objectives. On these terminals runs a main application software 27. The main application software 27 is principally comprised of a management function DLL (dynamic link library) 28, an examination operation DLL 29, a conference DLL 30, and a statistics/history DLL 31. And compressed images 36 can be temporarily stored in the hard disk 35.

Also, a compression-expansion unit 13 for image recording and compression is connected to the input/examination terminal 3 so as to enable the pickup of images in an examination. On this compression-expansion unit 13 runs a compression-expansion unit program 37, and compressed images 38 can be temporarily stored in memory therein. As mentioned already, the compression-expansion unit 13 is connected to the electronic endoscope apparatus 14.

Now a description will be given of information concerning examination, that is, the types of information to be managed by the present system. There are principally the following four types (1 to 4) of information managed by the present system.

### 1. Patient information

### (1) Basic patient information

This is information for identifying each individual patient, comprising "patient ID", "name", "birth date", "sex" and so forth. The "patient ID" is a number corresponding uniquely to a patient so as to identify the patient in distinction from the others.

### (2) Patient profile information

This is information representing the characteristics and conditions of a patient, which is comprised of "blood group", "height/weight", "allergy", "disorder", "infection", "disease & notabilia", "laboratory test results", "previous medication information" and so forth.

### 2. Examination request information

This is information concerning a request (order) for examination when a request for examination is made to the endoscopic department by any of the other hospital departments. The information includes order key information ("order No.", "order date", etc.), requester information ("requesting department name", "requesting physician name", "order date", etc.), order information ("requested disease name", "examination purpose", "examination type", "examination items", "examination regions", "comment", "schema images" (hereinafter also referred to as "schematic images"), etc.), examination booking information ("examination date", "implementation hours", etc.), and so forth. These units of information are sent to the department systems from an HIS (hospital information system) functioning as a management system. The order key information is information for uniquely identifying an examination order in distinction from the others.

### 3. Examination implementation information (accounting information)

This is information concerning the implementation of examination, comprising "implementation date and time", "implementer", "implementation place", "procedure", "chemicals", "equipment", and so forth. The information on the "procedure" performed, "chemicals" and "equipment" used, etc., is used in accounting. These units of information are sent from the system in the endoscopic department to the HIS, where they are processed by an accounting system therein.

### 4. Examination results information (report, report information)

This is information concerning the results of examination, that is, information that serves as a report in response to a request for examination. The information is comprised of "reporting date", "reporter", "diagnosis", "findings", "treatment", "comment", "post-examination notes and instructions", "images", "schema images (hereinafter also referred to as "schematic images"), and so forth. These units of information can be referenced not only at a medical image filing system in the endoscopic department, but also can be referenced at each department system after they are sent from the present system to the HIS. Accordingly, the results of examination (report) can be referenced in the department which has made a request for the examination.

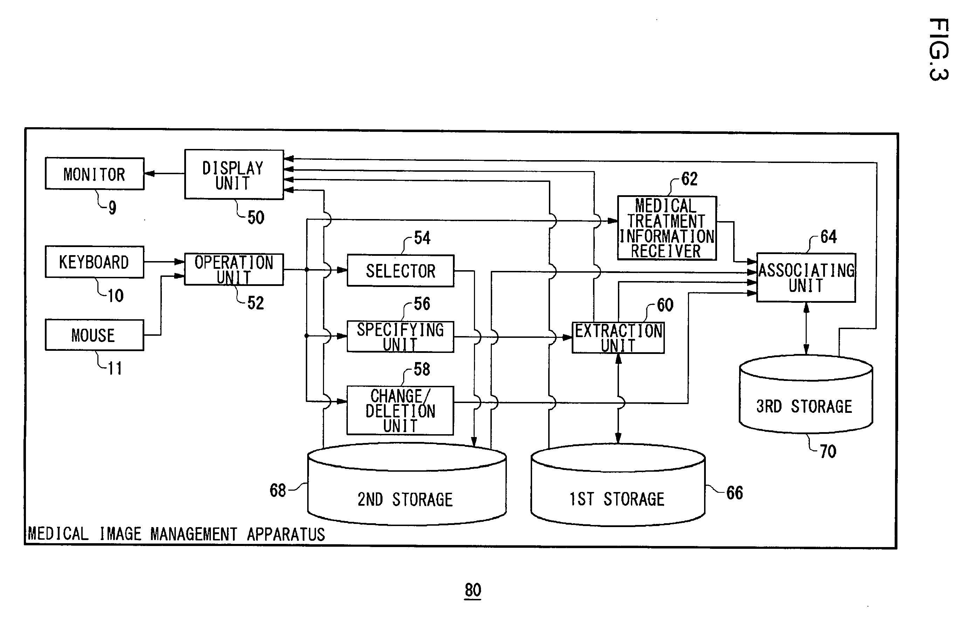

FIG. 3 illustrates a structure of a medical image management apparatus 80 which is included in an endoscopic examination management system. The medical image management apparatus 80 is equivalent to any of a reception terminal 2, an input/examination terminal 3 and a search terminal 4 as shown in FIG. 1 and FIG. 2, which includes a function for managing examination images and a function for preparing a report. The medical image management apparatus 80 includes a monitor 9, a keyboard 10, a mouse 11, a display unit 50, an operation unit 52, a selector 54, a specifying unit 56, a change/deletion unit 58, an extraction unit 60, a medical treatment information receiver 62, an associating unit 64, a first storage 66, a second storage 68, and a third storage 70.

The operation unit 52 outputs instructions received from the keyboard 10 and the mouse 11 to each component unit in the medical image management apparatus 80. The display unit 50 displays information received from the component units in the medical image management apparatus 80 on the monitor 9. The display unit 50 also adjusts the layout of display on the monitor 9.

The first storage 66 stores schema diagrams (schematic diagrams). Note that schema diagrams herein can mean either images displayed on the monitor 9 or image data, but no distinction will be made in the following description. The first storage 66 also stores positional information from the schema diagrams. As aforementioned, the positional information is information on coordinates defined on a schema diagram. Hence, a given position in a schematic diagram may be represented by coordinates on the X axis and Y axis, with the origin (0, 0) at the top-left position therein, for instance. That is, a schematic diagram is represented by a two-dimensional coordinate system. Further, the first storage 66 stores regional information corresponding to the positional information. Here the regional information is the information on the regions predefined in the entirety of the bronchial tubes. For example, it may be denoted in such a manner as "L B4" or "L B4a". Since the regional information is associated with the positional information, it is possible to identify a region by specifying an arbitrary position on a schematic diagram.

The second storage 68 stores examination images. Here it is assumed that there are a plurality of examination images. The examination images are obtained from the compression-expansion unit 13 connected to the electronic endoscope apparatus 14. The display unit 50 displays a schematic diagram stored in the first storage 66 and a plurality of examination images stored in the second storage 68 in their respective display regions. For example, a plurality of examination images are displayed in the left-hand region of the screen in a thumb-nail fashion, and a schematic diagram is displayed in the region to the right thereof.

The selector 54 selects an examination image to be associated with the schematic diagram out of the plurality of examination images displayed by the monitor 9 and the display unit 50, based on the instructions received via the operation unit 52. For example, the selector 54 selects an examination image according to the selection made by the mouse 11. The specifying unit 56 specifies a position on the schematic diagram displayed by the display unit 50, which is the position to be associated with the examination image selected by the selector 54, based on the instructions received via the operation unit 52. For example, the specifying unit 56 specifies a position on the schematic diagram where the selected examination image has been drag-and-dropped thereon.

The extraction unit 60 reads the coordinates in the position specified by the specifying unit 56. That is, the extraction unit 60 reads a drag-and-dropped position on the coordinates predefined in the schematic diagram. Also, the extraction unit 60 extracts the regional information associated with the specified position, based on the read coordinates and the positional information stored in the first storage 66. For example, the extraction unit 60 extracts uniquely a single unit of regional information from the read coordinates.

On the other hand, information on a plurality of neighboring regions may be combined together, and the regional information of this broader and generic conceptualization may be stored in the first storage 66 in association with the positional information. In such a case, the first storage 66 extracts generic regional information from read coordinates. Further, the extraction unit 60 displays information on a plurality of regions contained in the generic regional information via the display unit 50, and receives the selection of any of the information on the plurality of regions via the operation unit 52. As a result, the extraction unit 60 extracts the information on the selected region.

Note that when the extraction unit 60 cannot extract any regional information, the display unit 50 displays a message indicating the failure in extraction. For example, such a case may be when there is no regional information defined for a part of a schematic diagram, so that the regional information is not associated with the positional information stored in the first storage 66.

The associating unit 64 associates the regional information extracted by the extraction unit 60 with the examination image selected by the selector 54. The associating unit 64 also carries out association of positions specified by the specifying unit 56. Thus, the associating unit 64 associates regional information, positional information, and examination images with one another. The third storage 70 stores the association performed by the associating unit 64. The display unit 50 displays additional information indicating the association of an examination image on the schematic diagram image, based on the association with the position stored in the third storage 70. The additional information, which may be presented in any form, should take a form that will facilitate the user to recognize the fact of the examination image being associated. Such additional information allows the physician in charge to easily recognize where in the schematic diagram the examination image is located. Moreover, when certain regional information is identified, the display unit 50 displays the corresponding regional information in the vicinity of the examination image already being displayed.

Once the association between regional information and examination images is stored in the third storage 70, the medical treatment information receiver 62 instructs the physician in charge to input information on medical treatment via the display unit 50. Information on medical treatment meant here is equivalent to the aforementioned findings and the like. It is to be noted also that the information on medical treatment is in correspondence to the associated regional information. Also, the medical treatment information receiver 62 receives information on medical treatment via the operation unit 52. The associating unit 64 associates information on medical treatment with regional information, and the third storage 70 stores the information on medical treatment associated with the regional information also. Further, the display unit 50 displays the information on medical treatment stored in the third storage 70 in a region thereof separate from the region where a schematic diagram and a plurality of examination images are being displayed.

The change/deletion unit 58 receives an operation of change or deletion relative to the additional information displayed by the display unit 50. Here the change/deletion unit 58 receives such operation via the operation unit 52. Upon receipt of the operation, the change/deletion unit 58 changes or deletes the association stored in the third storage 70 accordingly. It should be noted here that a position change can also be made by drag-and-drop to an object indicating the additional information. In so doing, there will be a change in the regional information assigned to the object indicating the additional information, so that it will be necessary to verify the relationship to the findings or the like so far assigned. Thus, after the display unit 50 has displayed a verification dialog indicating a required change, the associating unit 64 cancels the foregoing association and carries out the new association. As a result, the findings or the like, as well as the examination images, are assigned to the new regional information.

In terms of hardware, this structure described as above can be realized by a CPU, a memory and other LSIs of an arbitrary computer. In terms of software, it can be realized by memory-loaded programs or the like, but drawn and described herein are function blocks that are realized in cooperation with those. Hence, it is understood by those skilled in the art that these function blocks can be realized in a variety of forms such as by hardware only, software only or the combination thereof.

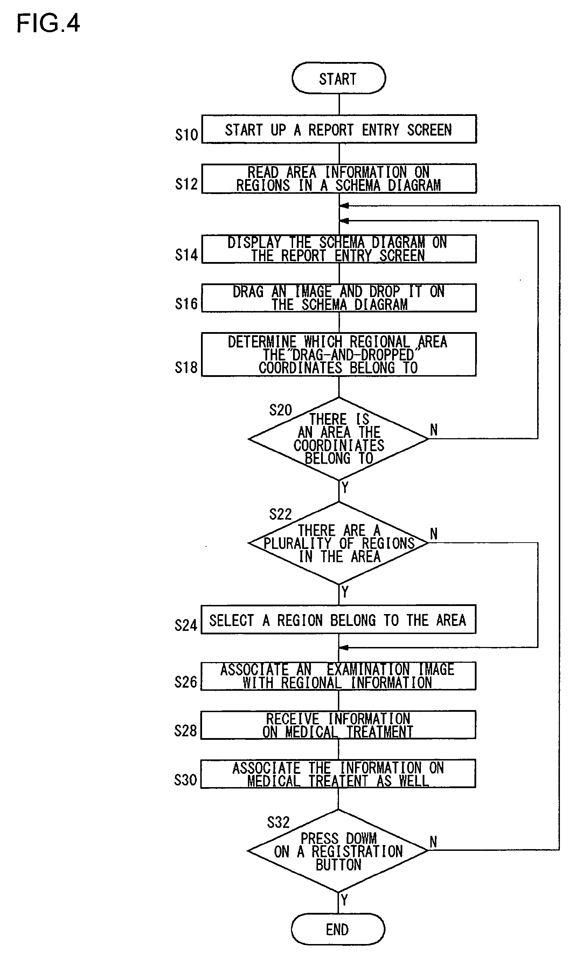

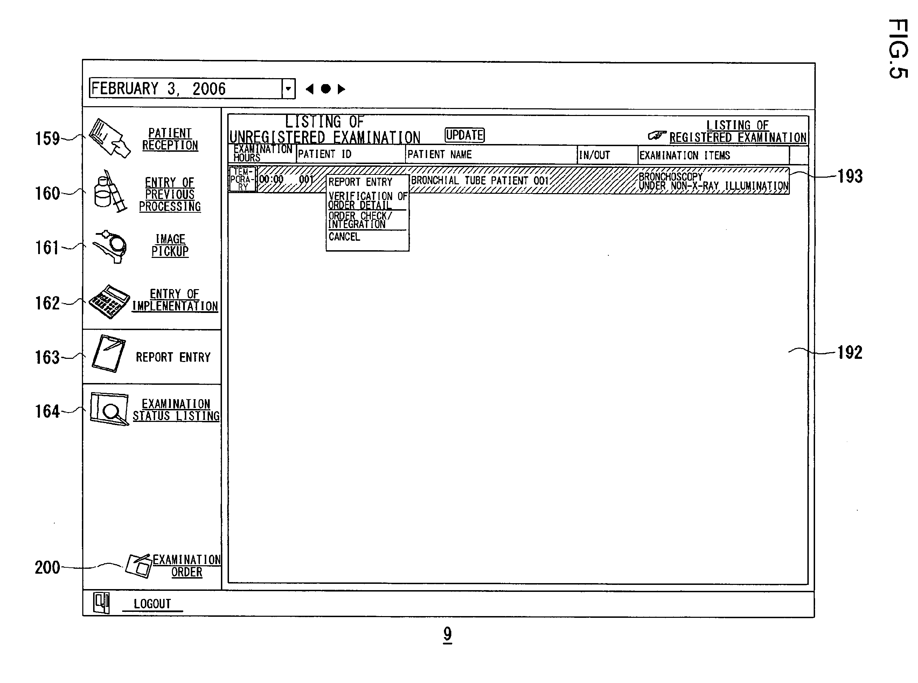

A description will be given of an operation of a medical image management apparatus 80 implementing the structure as described above. FIG. 4 is a flowchart showing a procedure for managing medical images by the medical image management apparatus 80. The display unit 50 starts up a report entry screen (S10). FIG. 5 illustrates a report selection screen displayed on the monitor 9 for selecting a type of report to be inputted. Displayed on the left-hand side of the screen are a patient reception icon 159, a previous treatment entry icon 160, an image pickup icon 161, an implementation entry icon 162, a report entry icon 163, an examination status listing icon 164, and an examination order button 200. One of these icons is selected with a click of the mouse 11 by a physician in charge, and a program for the selected icon runs. Here it is assumed that the report entry icon 163 has been selected. Also, in the report unregistered examination listing screen 192, an examination listing 193 is displayed, and a new window is displayed for "Patient ID: 001". With a click on "report entry", the first screen of the report entry is started up. Refer back to FIG. 4.

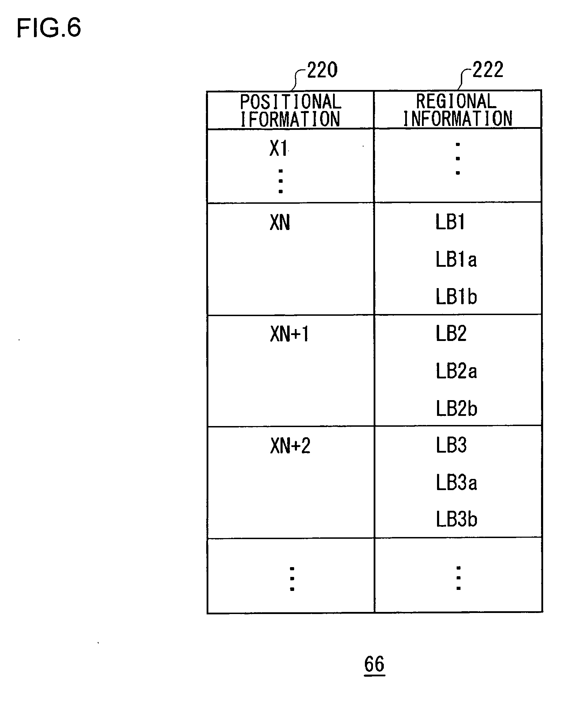

The extraction unit 60 reads area information on regions in a schematic diagram from the first storage 66 (S12). FIG. 6 shows a data structure of positional information and regional information stored in the first storage 66. The data structure is composed of a positional information column 220 and a regional information column 222. Stored in the positional information column 220 are coordinates, such as "X1". Here, "X1" may correspond to a single coordinate point (x0,y0) or to an area enclosed by four points (x0,y0), (x1,y0), (x1,y1), and (x0,y1). Assume here that x0<x1 and y0<y1 hold. Note that the area is not limited to a rectangle. Stored in the regional information column 222 is at least one unit of regional information associated with one unit of positional information. The regional information is denoted by "L B1a", for instance. Refer back to FIG. 4.

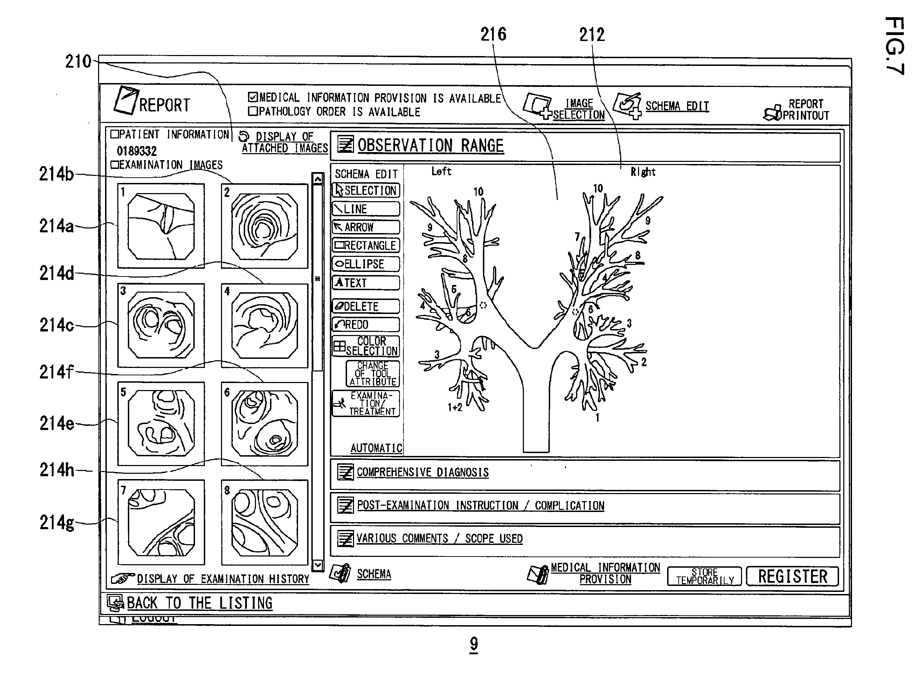

The display unit 50 displays a schematic diagram on the report entry screen (S14). FIG. 7 illustrates a report entry screen displayed on the monitor 9. The screen is provided with a first area 210 and a second area 212. Displayed in the first area 210 are a first examination image 214a through an eighth examination image 214h, which are generically referred to as examination images 214. The examination images 214 are stored in the second storage 68. Displayed in the second area 212 is a schematic diagram 216. The schematic diagram 216 is stored in the first storage 66. As previously indicated, coordinates are defined on the schematic diagram 216, and all the parts of the schematic diagram 216 are associated with their respective units of positional information. Also, regional information is defined in association with the positional information.

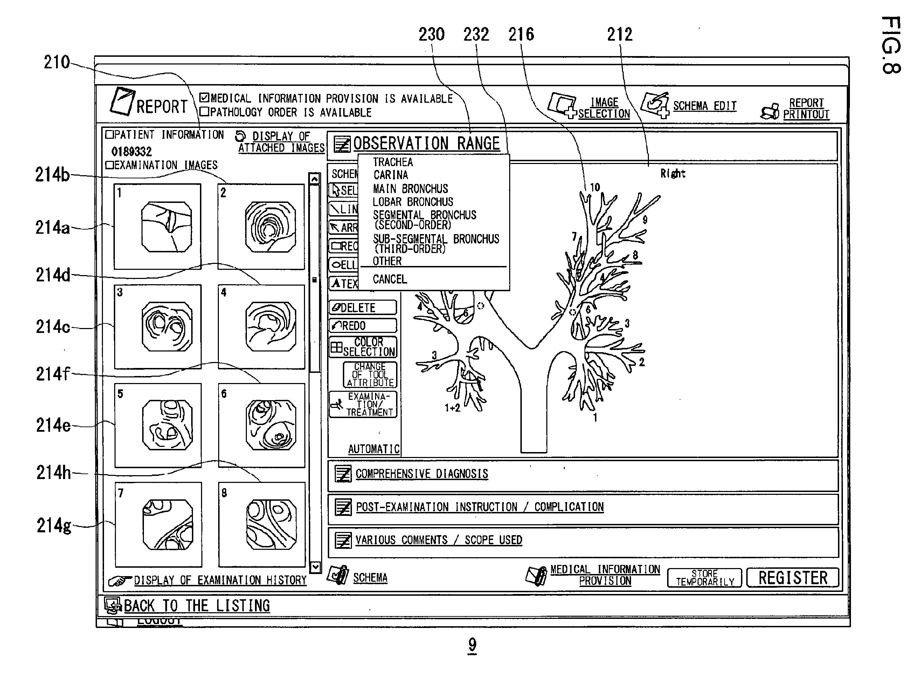

FIG. 8 illustrates an observation range setting screen displayed on the monitor 9. When a physician in charge clicks an observation range button 230 using the mouse 11, an observation range window 232 is displayed. Clicking one of the entries, such as "Trachea", in the observation range window 232 causes the observation range to be inputted. Refer back to FIG. 4.

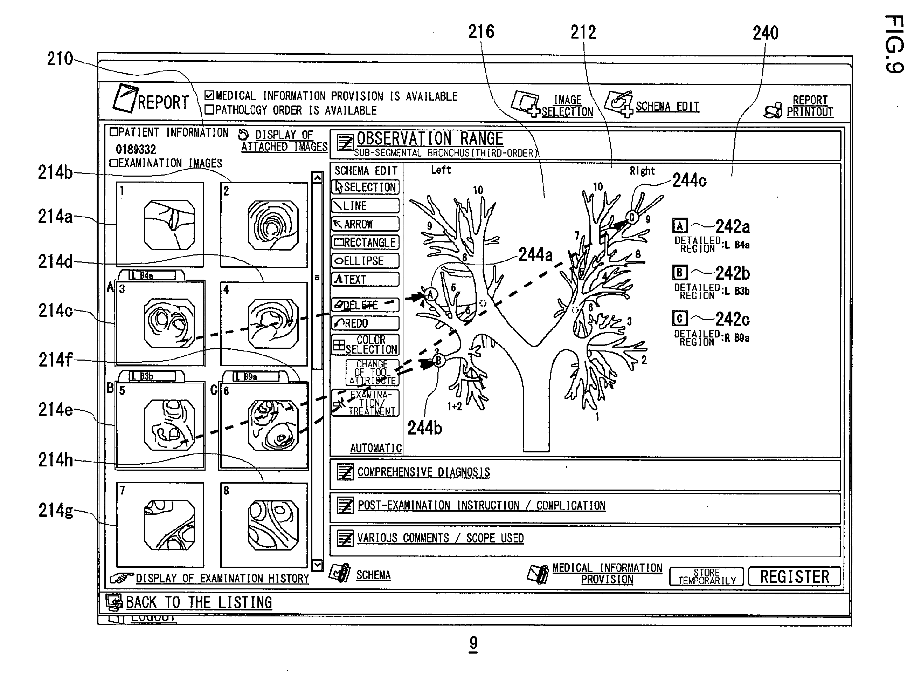

The physician in charge drags an examination image and drops it on the schematic diagram by using the mouse 11 (S16). FIG. 9 illustrates a detailed region setting screen displayed on the monitor 9. The physician in charge performs the dragging by holding down the right click button on a given examination image 214. Also, the physician in charge performs the dropping by releasing the right click button at a target point on the schematic diagram 216. The operation as described above corresponds to the operation by the selector 54 and the specifying unit 56. For example, a third examination image 214c is dragged and dropped on the schematic diagram 216 following the arrow of the broken line. The drag-and-dropped position is displayed as a first additional information 244a. Also, there is a tab added to a third examination image 214c, and the tab shows regional information which is associated by a processing to be described later.

And a similar processing is done for a fifth examination image 214e and a sixth examination image 214f as well, and in consequence, second additional information 244b and third additional information 244c are displayed respectively on the schematic diagram 216. Further, simultaneously with the drag-and-dropping of examination images 214 on the schematic diagram 216, a first medical treatment information icon 242a to a third medical treatment information icon 242c, which are generically referred to as medical treatment information icons 242, are displayed in a third area 240. The medical treatment information icons 242 are used when information on medical treatment, such as findings and the like, is to be entered. It is to be noted that the selector 54 forbids the drag-and-dropping of examination images 214 in areas where detailed regions are not assigned on the schematic diagram 216. Also, the display unit 50 indicates to the user by a message screen that such a drag-and-dropping cannot be performed. In this regard, the display unit 50 may indicate the message visually to the user by using different colors for areas of the schematic diagram 216 where detailed regions are assigned and areas where they are not. Refer back to FIG. 4.

The specifying unit 56 and the extraction unit 60 determines which regional area the "drag-and-dropped" coordinates belong to (S18). If there is no area the coordinates belong to (N of S20), the operation returns to Step 14. At this time, the display unit 50 may display the aforementioned message indicating the inability to extract. For example, such a message as "No applicable regional information" or "No drag-and-dropping in this area" may be displayed. If there is an area the coordinates belong to (Y of S20) and if there are a plurality of regions in the area (Y of S22), then the extraction unit 60 will select a region belonging to the area according to the instruction from the operation unit 52 (S24). Here, the case where there are a plurality of regions in the area corresponds to the case where there are plural units of regional information relative to a single unit of positional information as shown in FIG. 6.

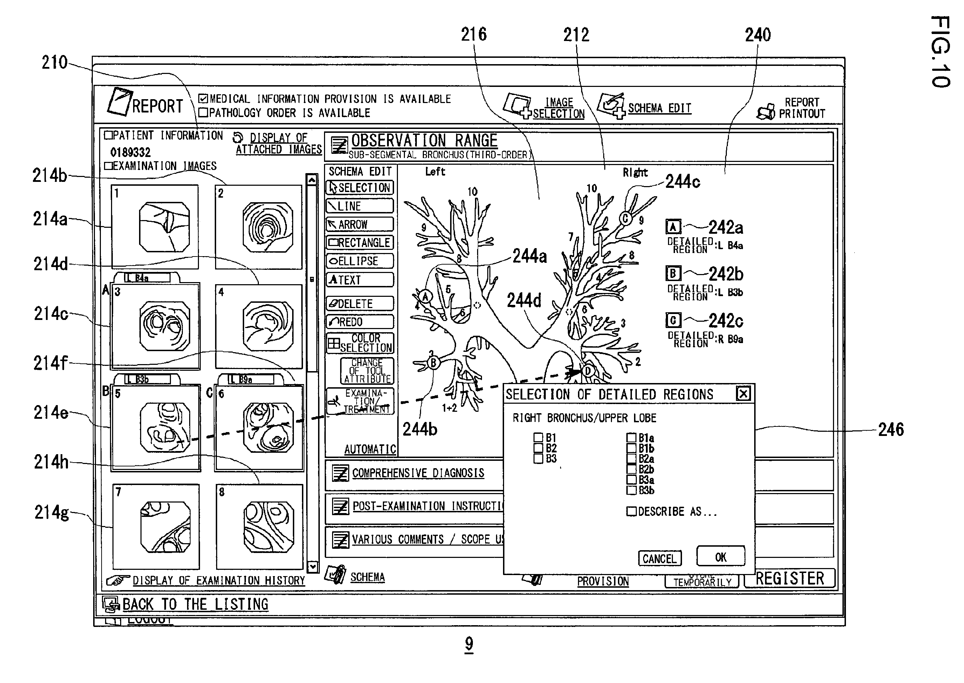

FIG. 10 illustrates a verification dialog for a detailed region displayed on the monitor 9. A detailed region selecting window 246 is displayed, and names of currently selectable regions are shown in the detailed region selecting window 246. As the physician in charge selects one of the regions shown in the detailed region selecting window 246, the one corresponding unit of regional information is selected. At this time, the extraction unit 60 selects information on regions present in the neighborhood from the plural units of regional information according to the drag-and-dropped position and displays them in the detailed region selecting window 246. For example, the extraction unit 60 selects regional information associated with the positional information shown in FIG. 6. Refer back to FIG. 4.

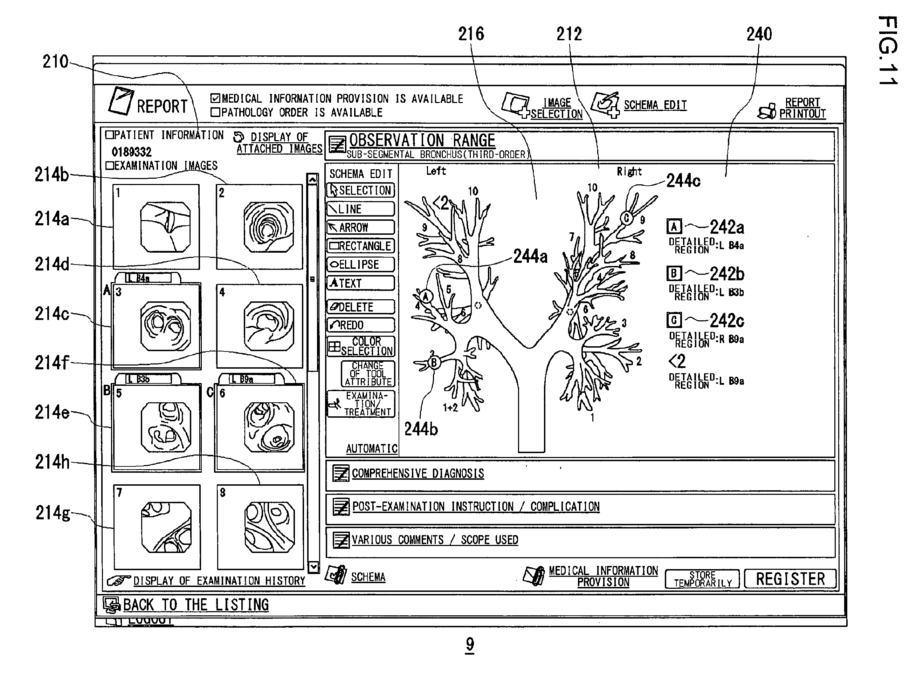

On the other hand, if there are not a plurality of regions in the area (N of S22), Step 24 is skipped. The associating unit 64 associates an examination image with the regional information (S26). The medical treatment information receiver 62 receives information on medical treatment via the operation unit 52 (S28). FIG. 11 illustrates a report entry screen displayed on the monitor 9. There is an indication of "<2" on the schematic diagram 216. This indication is an "examination/processing mark", and as the physician in charge clicks the "examination/processing mark" or additional information 244, a screen is displayed for entry of the information on medical treatment. Through this procedure, the medical treatment information receiver 62 starts up a screen for instructing the input of information on medical treatment. Note that this screen can also be started by a click on the medical treatment information icon 242 by the physician in charge.

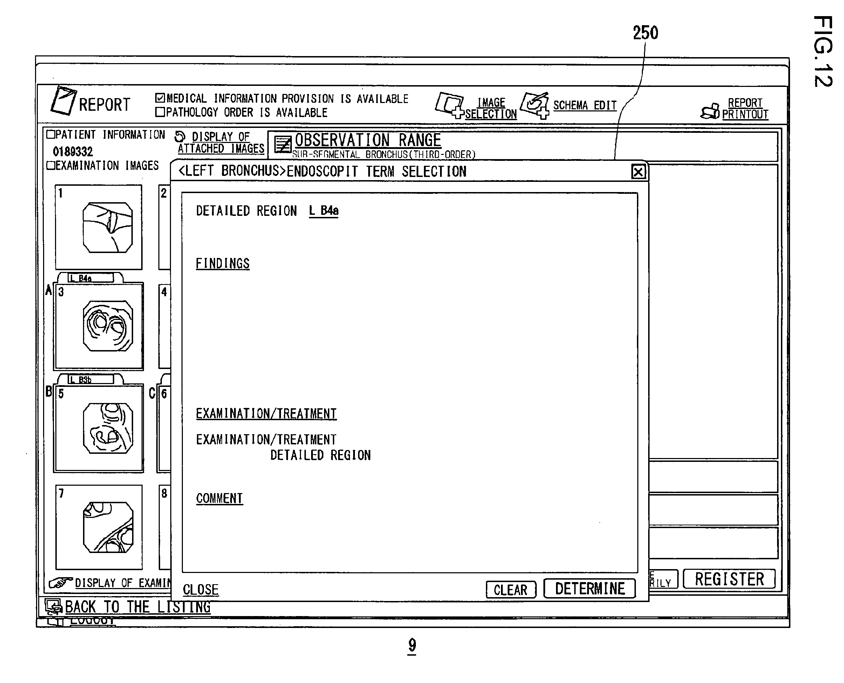

FIG. 12 illustrates another report entry screen displayed on the monitor 9. The screen shown in FIG. 12 is equivalent to one for inputting information on medical treatment. An endoscopic term selecting window 250 is displayed, and a previously-inputted code is displayed for detailed region. Also, findings, examination/treatment, and comment may be entered in the endoscopic term selecting window 250. And with the "Determine" button pressed, the contents entered in the endoscopic term selecting window 250 are determined. Refer back to FIG. 4.

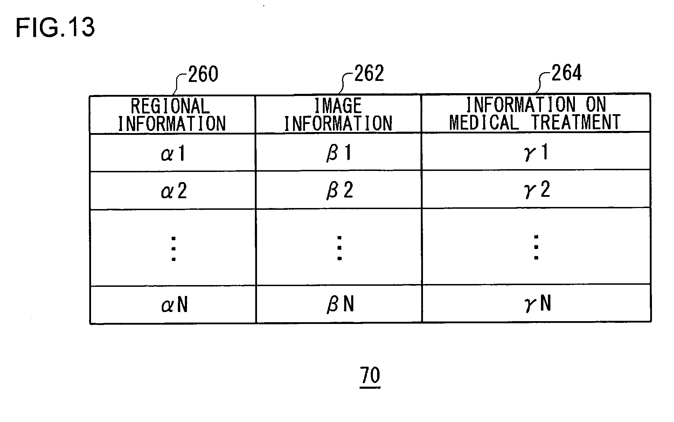

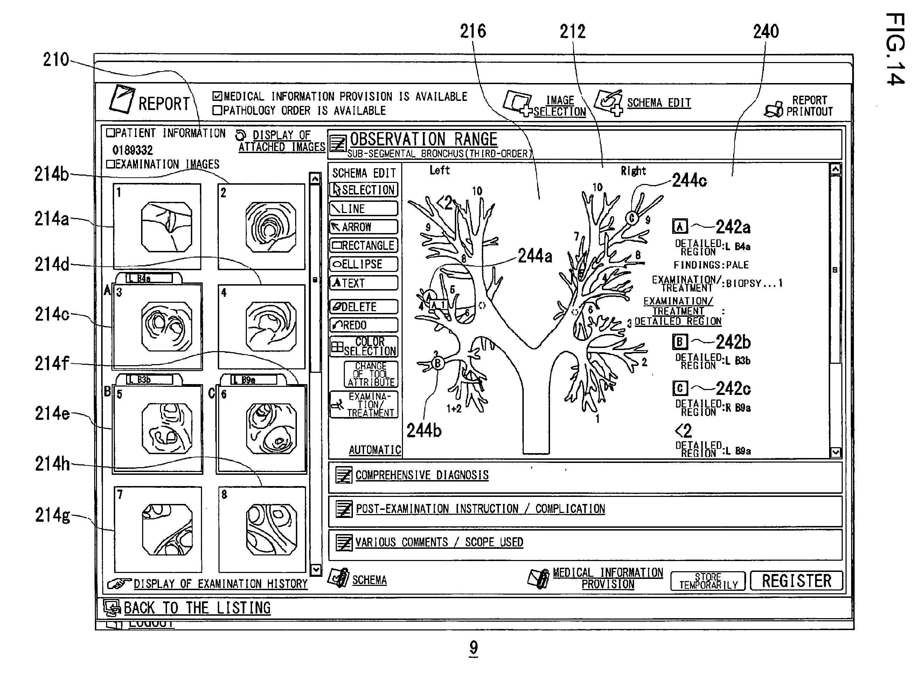

The associating unit 64 also associates the information on medical treatment (S30). FIG. 13 shows a data structure for association stored in the third storage 70. The structure includes a regional information column 260, an image information column 262, and a medical treatment information column 264. For example, regional information "α1", image information "β1", and medical treatment information "γ1" are associated with one another. Note that plural units of image information may be associated with a single unit of regional information. FIG. 14 illustrates a completed screen after data entry displayed on the monitor 9. When examination/treatment has been done to a plurality of regions other than the region (detailed region) for findings, entry must be made for "Examination/treatment detailed region". There are two methods for actual entry. In one method, "Examination/treatment detailed region" below the first medical treatment information icon 242a must be clicked. In the other, an applicable position in the schematic diagram 216 must be clicked.

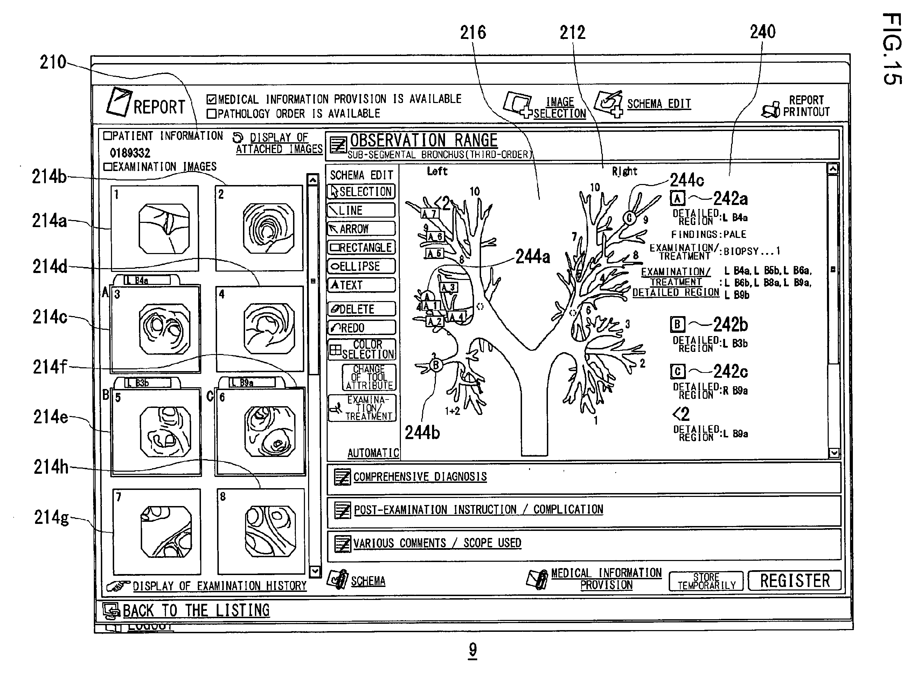

FIG. 15 illustrates another completed screen after data entry displayed on the monitor 9. A code, such as "A 1" is displayed at a position in the schematic diagram 216 clicked by the physician in charge. With the display of such a code, a corresponding examination/treatment detailed region is displayed. Note that a detailed region corresponds to regional information on the position picked up in an examination image 214 by an endoscope whereas an examination/treatment detailed region corresponds to regional information on the region picked up in the examination image 214. Although generally there is difference between the image-taking position and the position picked up in the image, their respective positions may be shown definitively by an arrangement to make the entry of both possible. Refer back to FIG. 4. If the registration button is not pressed (N of S32), a return is made to Step 14. If, on the other hand, the registration button is pressed (Y of S32), the processing is completed.

In respect of report preparation, the operation of a medical image management apparatus 80 may also be described as follows. In the preparation of a report, the display unit 50 displays a schematic diagram 216 on the report entry screen according to the instruction from the user preparing the report. The specifying unit 56 specifies a region on which information on medical treatment is to be inputted, in the displayed schematic diagram 216. The display unit 50 displays information concerning the specified region as the object of input. The medical treatment information receiver 62 prompts an input of information on medical treatment for the displayed object of input. The associating unit 64 prepares a report based on the inputted information on medical treatment.

Modifications to the present exemplary embodiments will be described hereinbelow. As described already, examination image marks are attached to a schematic diagram on an endoscopic electronic chart. If two or more examination image marks are attached to the same part of the schematic diagram, additional information 244, or the examination image marks, will be displayed overlapping each other. In such a case, a problem to be addressed is how the superimposed examination image mark is moved away so that the one below can be displayed. And when the superimposed examination image mark is moved away, another problem is that the examination image mark is now dislocated from the original position. In this modification, therefore, all the examination image marks displayed overlapping each other on a hint screen can be displayed separately simply by bringing a mouse cursor close to a representative examination image mark, thus making it unnecessary to move them away from each other.

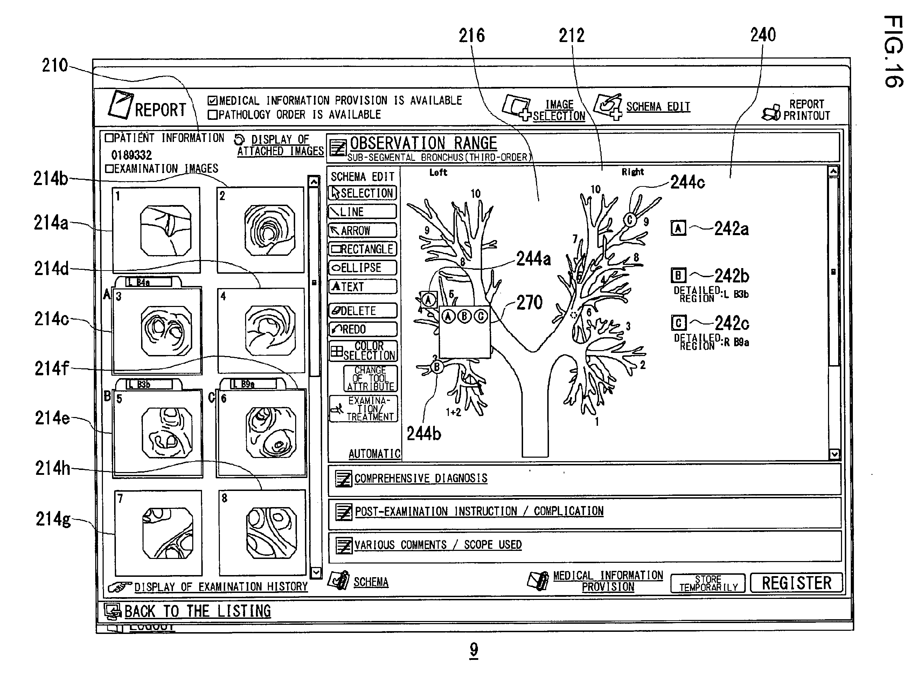

FIG. 16 illustrates still another completed screen after data entry displayed on the monitor 9. A plurality of examination images are associated with the position where first additional information 244a is displayed. As shown, however, a single unit of first additional information 244a is displayed. When the cursor of the mouse approaches the first additional information 244a, the display unit 50 has a pop-up window 270 displayed. Also displayed in the pop-up window 270 are three examination images which are associated with this position. Thus the pop-up window 270 allows the physician in charge to recognize the examination images associated therewith.

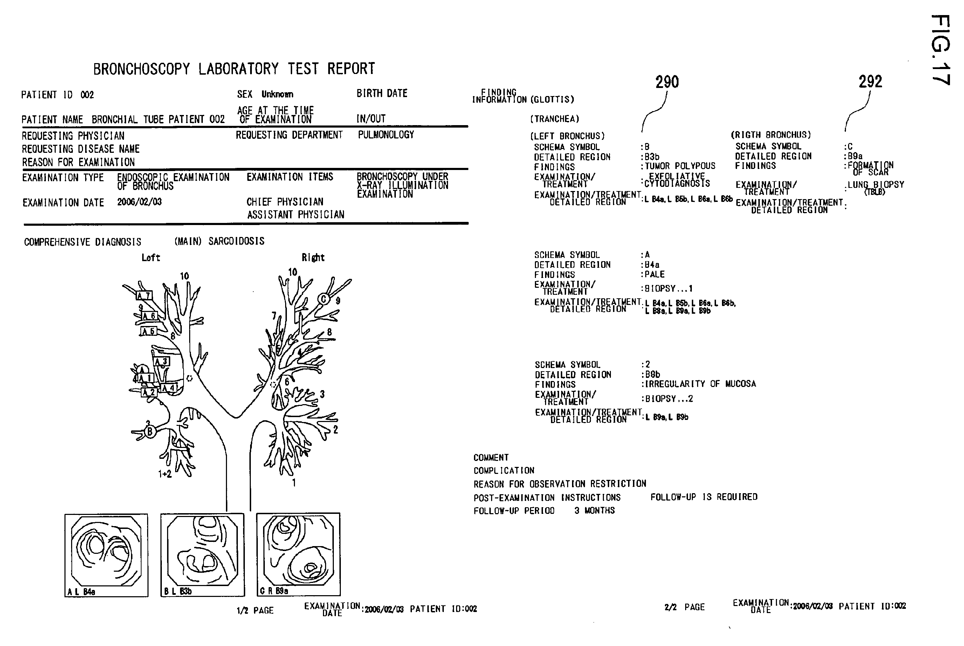

FIG. 17 illustrates a report prepared by a medical image management apparatus 80. Shown in FIG. 17 is a body of information that has been associated by processing thus far described. Note that a report like this can be prepared by the extracting unit 60. For "findings information", in particular, a left bronchial tube column 290 and a right bronchial tube column 292 are provided. In the left bronchial tube column 290, information concerning the left bronchial tube is displayed, whereas, in the right bronchial tube column 292, information concerning the right bronchial tube is displayed.

According to the exemplary embodiments of the present invention, positional information and regional information are associated with each other in advance before they are stored, so that examination images can be easily associated with the regional information by simply specifying a position on a displayed schematic diagram as the position with which the examination images are to be associated. Also, visibility is fairly improved since additional information indicating the association of examination images is displayed on the schematic diagram. Moreover, change or deletion processing can be performed quite easily since the stored association can be changed or deleted simply by changing or deleting the additional information. Also, a message indicating an inability to extract is displayed, so that it is possible to instruct the user to perform the processing correctly. Also, examination images and information on medical treatment may be associated with a single unit of regional information. Further, processing is simplified because regional information when inputting examination images and regional information when inputting information on medical treatment can be identified using a shared system.

Also, a report may be prepared easily since information on medical treatment and a schematic image can be associated with each other easily. Moreover, the regional information can be registered by simply selecting and drag-and-dropping an examination image on a schematic diagram. On an endoscopic electronic chart, the registration of a region and the registration of a part of a schematic diagram corresponding to the registered region are carried out in a single step, so that the registration of a region can be simplified. Also, on an endoscopic electronic chart, all the examination image marks displayed overlapping each other on a hint screen can be displayed separately by bringing a mouse cursor close to a representative examination image mark, thus making it unnecessary to move them away from each other.

The present invention has been described based on the exemplary embodiments. These embodiments are merely exemplary, and it is understood by those skilled in the art that various modifications to the combination of each component and each process thereof are possible and that such modifications are also within the scope of the present invention.

In an exemplary embodiment, a schematic diagram 216 and examination images 214 for the medical image management apparatus 80 correspond to an endoscopic examination of the bronchial tubes. However, the arrangement is not limited thereto, and the medical image management apparatus 80 may process examination images 214 which are acquired by an examination other than an endoscopic examination of the bronchial tubes. Also, a schematic diagram showing some other related information may be utilized. Such a schematic diagram showing some other related information is a schematic diagram, for instance, where information on a lymph node on the bronchial tube is displayed.

While the preferred embodiments of the present invention have been described using specific terms, such description is for illustrative purposes only, and it is to be understood that changes and variations may be made without departing from the spirit or scope of the appended claims.

## Claims

1. A medical image management apparatus (80), comprising:
a first storage (66) which stores a first medical image, positional information in the first medical image and regional information corresponding to the positional information, respectively;
a second storage (68) which stores a second medical image different from the first medical image stored in said first storage (66);
a display unit (50) which displays the first medical image stored in the first storage (66) and the second medical image stored in the second storage (68) in separate areas, respectively;
a selector (54) which selects a second medical image to be associated with the first image from among second images displayed on said display unit (50);
a specifying unit (56) which specifies a position to be associated with the second medical image selected by said selector (54) wherein the position is a position on the first medical image displayed on said display unit (50);
an extraction unit (60) which extracts regional information associated with the specified position, based on the position specified by said specifying unit (56) and the positional information stored in said first storage (66); and
a third storage (70) which stores association between the positional information extracted by said extraction unit (60) and the second medical image selected by said selector (54).

2. A medical image management apparatus (80) according to Claim 1, wherein said third storage (70) further stores association among the position specified by said specifying unit (56), the extracted positional information and the selected second medical image,
wherein based on the positional association stored in said third storage (70) said display unit (50) displays additional information, indicating that the second medical image is associated therewith, on the first medical image.

3. A medical image management apparatus (80) according to Claim 2, further comprising a change/deletion unit which changes or deletes the association stored in said third storage (70) by specifying an operation of changing or deleting the additional information displayed on said display unit (50).

4. A medical image management apparatus (80) according to Claim 1, wherein when said extraction fails to extract region information, said display unit (50) displays a message indicating that extraction has failed.

5. A medical image management apparatus (80) according to any one of Claim 1 to Claim 4, wherein said third storage (70) further stores information on a medical treatment associated with the regional information,
wherein said display unit (50) displays the information on a medical treatment stored in said third storage (70) in an area different from the area in which the first medical image and the second medical image are displayed.

6. A method for managing medical images, the method comprising:
displaying a first medical image and a second medical different from the second image on separate areas, respectively;
selecting a second medical image to be associated with the first image from among second images displayed;
specifying a position to be associated with the selected second medical image wherein the position is a position on the first medical image displayed;
extracting regional information associated with the specified position, based on the positional information, by referring to the positional information stored beforehand and regional information corresponding to the positional information; and
associating the extracted regional information with the selected second medical image.

7. A method for managing medical images, the method **characterized in that** regional information associated beforehand with positional information specified on a displayed schematic image and an examination image to be compared with the regional information are stored in a recording medium in a manner such that the regional information and the examination image are associated with each other.

8. A method for preparing a report, comprising:
displaying a schematic image on a report entry screen, based on a specification from a person who prepares the report;
specifying a region, where information on a medical treatment is to be inputted, on the displayed schematic image;
displaying information on the specified region, as information to be inputted;
prompting an input of the information on a medical treatment to the displayed information to be inputted; and
preparing the report based on the inputted information on a medical treatment.
